(19) Europäisches Patentamt
European Patent Office
Office européen des brevets

(11) Publication number: **0 194 097 B1**

(12) **EUROPEAN PATENT SPECIFICATION**

(45) Date of publication of patent specification: **25.04.90**

(51) Int. Cl.[5]: **A 61 K 7/50,** A 61 K 7/00

(21) Application number: **86301363.7**

(22) Date of filing: **26.02.86**

(54) **Mild cleansing mousse.**

(30) Priority: **01.03.85 US 707308**

(43) Date of publication of application:
**10.09.86 Bulletin 86/37**

(45) Publication of the grant of the patent:
**25.04.90 Bulletin 90/17**

(84) Designated Contracting States:
**AT BE CH DE FR IT LI LU NL SE**

(56) References cited:
**EP-A-0 106 193**
**DE-A-2 811 010**
**DE-A-2 841 377**
**FR-A-2 496 458**
**GB-A-2 103 236**
**US-A-3 959 160**
**US-A-4 438 095**
**US-A-4 491 539**

(73) Proprietor: **THE PROCTER & GAMBLE COMPANY**
**One Procter & Gamble Plaza**
**Cincinnati Ohio 45202 (US)**

(72) Inventor: **Schmidt, Robert Raymond**
**504 General Drive**
**Ft. Wright, KY 41011 (US)**
Inventor: **Fortna, Raymond Harry**
**11909 Balckhawk Circle**
**Cincinnati Ohio 45240 (US)**
Inventor: **Beyer, Harold Henry**
**7517 Golf Green Drive**
**Cincinnati Ohio 45242 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 194 097 B1

**Description**

The present invention is related to mild personal skin cleansers. More particularly, this invention relates to pressurized aerosol mousse dispensers of skin cleansers comprising surfactants and other cleansing aids.

In pressurized foam dispensers of the above-mentioned type, a foamable concentrate, generally an aqueous soap solution, is contained in a dispenser equipped with a dispensing head and valve, and pressurized with a normally gaseous propellant, e.g., a low molecular weight hydrocarbon or hydrocarbon mixture or a halohydrocarbon or halohydrocarbon mixture. Upon discharge of the emulsion through the dispensing head the volatilization of the dispersed liquid droplets of propellant causes the dispensed concentrate to foam. Depending upon the precise formulation of the concentrate, the dispensed product may range from a dense creamy foam to a light foam.

The term "emulsion" will be used throughout this specification and claims to refer to the whole liquid contents of the dispenser, i.e., the foamable concentrate plus liquid phase propellant, and the term "concentrate" will be used to refer to the liquid contents of the dispenser, other than the propellant, "liquid" in this context embracing solutions, emulsions and suspensions. In other words, the concentrate itself may be an emulsion or suspension and not necessarily a solution of the foam producing ingredients in a suitable liquid medium, which, in the case of the present invention, will be water. The term "mousse", as used herein, is the same as foam, and refers to the dispensed product unless otherwise specified.

The cleaning of skin with surface-active cleaning preparations has become a focus of great interest. Many people wash and scrub their skin with various surface-active preparations several times a day. Ideal skin cleansers should cleanse the skin gently, causing little or no irritation without defatting and overdrying the skin or leaving it taut after frequent routine use. Most lathering soaps, liquids and bars included, fail in this respect.

Certain synthetic surfactants are particularly mild. However, a major drawback of most mild synthetic surfactant systems when formulated for skin cleansing is poor lather performance compared to the highest bar soap standards (bars which are rich in coconut soap and superfatted). On the other side, the use of known high sudsing anionic surfactants with lather boosters can yield acceptable lather volume. Unfortunately, however, the highest sudsing anionic surfactants are, in fact, poor in clinical skin mildness. Surfactants that are among the mildest, such as sodium lauryl glyceryl ether sulfonate, (AGS), are marginal in lather. These two facts make the surfactant selection, the lather and skin feel benefit formulation process a delicate balancing act.

Moisturizers provide skin conditioning benefits. E.g., it is known that glycerin is added to bars and liquid cleansing products for skin benefits. Glycerin in liquids at levels of greater than 8% is extremely difficult to lather and in nonsoap bars is difficult to process. Glycerin has also been used in a soap based shaving cream at a 10% level.

US—A—3,959,160 discloses aerosol shaving foam compositions which comprise soaps or nonsoap anionic surfactants and fatty alcohols. The Horsler et al. examples include up to 5% glycerin. This patent does not disclose higher levels of moisturizer or polymeric skin feel aids.

Examples of mild liquid cleansing compositions are disclosed in the following references. US—A—4,338,211 discloses liquid skin cleanser with 2.3% to 3% AGS, the polymer JR400 and small amounts of free fatty acid plus a fatty acid alkylolamide as lather boosting agents. Compositions containing the surfactants AGS, betaine and sacrosinate are not disclosed.

US—A—4,491,539 discloses liquid cleansing products comprising about 5% to 30% of surfactant, about 0.1% to about 1.0% of guar material, about 0.15% to about 1.0% of nonionic carboxyvinyl polymer, and water. Exemplary compositions containing the surfactants AGS, betaine and sarcosinate or high levels of moisturizer are not disclosed. GB—A—2,103,236 discloses a light duty, liquid detergent containing guar gum, a ternary surfactant mixture including betaine. AGS is not used. GB—A—2,114,995A discloses a cleansing product based on acylisethionates and cationic polymers. FR—A—2496458 discloses liquid phase mousse compositions, optionally in aerosol form, comprising an oil which is liquid at room temperature, an oil-soluble surfactant, a cationic polymer material and water.

Rather stringent requirements for skin cleansers limit the choice of surface-active agents, and final formulations represent some degree of compromise. Mildness is often obtained at the expense of effective cleansing, or lathering may be sacrificed for either mildness, product stability, or both.

None of the above-cited prior art formulations contain the composition of mild synthetic surfactant, high level of moisturizer with polymer skin feel aids.

Therefore, one object of this invention is the development of skin cleaning compositions which exhibit desired skin feel after washing together with effective cleansing through surface activity and abundant rich, creamy foam. The desired skin feel is achieved through the combined action of mild surfactants, skin feel polymer and high levels of moisturizers which act together to leave the skin feeling less taut/dry, more moisturized, softer and smoother after washing.

Other objects will become apparent from the detailed description below.

Accordingly, the invention provides a skin-cleansing mousse-forming product having a pressurized dispenser equipped with a dispensing head and valve and containing therein a foam-forming emulsion comprising:

a. 88% to 97% by weight of the emulsion of a concentrate containing by weight of the concentrate:

1. from 3% to 20% of a nonsoap surfactant selected from alkyl glyceryl ether sulfonates (AGS), anionic acyl sarcosinates, methyl acyl taurates, N-acyl glutamates, alkyl glucosides, acyl isethionates, alkyl sulfosuccinates, alkyl phosphate esters, ethoxylated alkyl phosphate esters, trideceth sulfate, methyl glucose esters, protein condensates, mixtures of ethoxylated alkyl sulfates and alkyl amine oxides, betaines, sultaines, and mixtures thereof;

2. a polymeric skin feel aid at a level of from 0.05% to 5%;

3. a nonocclusive moisturizer at a level of from 10% to 60%, the moisturizer being selected from:

a) water-soluble liquid polyols, said polyols being selected from glycerin, polyethylene glycol, propylene glycol, sorbitol, polyethylene glycol and propylene glycol ethers of methyl glucose, polyethylene glycol and propylene glycol ethers of lanolin alcohol, and mixtures thereof;

b) essential compounds found naturally occurring in the stratum corneum of the skin, said essential compounds being selected from sodium pyrrolidone carboxylic acid, lactic acid, urea, L-proline, guanidine, pyrrolidone, and mixtures thereof;

c) water-soluble nonpolyol nonocclusives selected from hexadecyl, myristyl, isodecyl or isopropyl esters of adipic, lactic, oleic, stearic, isostearic, myristic or linoleic acids, sodium isostearoyl-2-lactylate, sodium capryl lactylate, hydrolyzed protein, aloe vera gel and acetamide MEA (N-acetylethanolamine), and mixtures thereof;

4. water; and

b. from 3% to 12% by weight of the emulsion of a propellant.

The concentrate of the present invention preferably contains from 15% to 87% water, more preferably 35% to 75% water.

The invention relates to a skin cleaning mousse with superior after washing skin feel combined with rich, creamy foam. This mild cleansing mousse composition is believed to provide less skin irritation and facial tautness than commercially available toilet soap bars, synthetic toilet bars or other known specialty lathering facial cleansing products. The mousse of this invention also provides abundant, rich creamy foam and leaves the skin feeling soft and smooth after washing.

The preferred mousse concentrate contains: 15—40% glycerin; 6—12% alkyl glyceryl ether sulfonate (AGS) plus a co-surfactant selected from (i) anionic alkyoyl (acyl) sarcosinate or (ii) amphoteric betaine or sultaine, or mixtures thereof; and 0.1—1% polymeric skin feel aid selected from cationic polymers including guar gums, cellulosic resins; homopolymers and copolymers of dimethyldiallylammonium chloride and nonionic guar gums.

Moisturizers/emollients

Moisturizers are included to provide the skin conditioning benefits and to aid in leaving the skin feeling less taut/dry, more moisturized, softer and smoother after washing. The aerosol product form surprisingly allows very high levels of the moisturizers to be incorporated without adversely affecting the foam-forming ability of the product or its in-use feel, thereby delivering better after washing skin feel than heretofore available from conventional lathering liquid cleansers.

The moisturizers useful in the present invention are used at a level of 10% to 60% by weight of the concentrate. The preferred levels of moisturizers are, respectively, 12% to 40% and 15% to 40%. The preferred moisturizers are the nonocclusive liquid water-soluble polyols and the essential compounds found naturally in the skin. The most preferred moisturizer is glycerin. The moisturizer, at these levels, provides superior after washing skin feel, less taut, more moisturized, softer and smoother. The mousse of the present invention is surprisingly stable. In the mousse formulation of this invention the high level of moisturizer can also provide an enhanced creamy foam.

The term "moisturizer" is often used within the cosmetic industry without very exact definition. The term is sometimes used as synonymous with emollient, and is then meant to describe a material which imparts a smooth and soft feeling to the skin surface.

There are two ways of reducing water loss from the stratum corneum. One is to deposit on the surface of the skin an occlusive layer which reduces the rate of evaporation. The second method is to add nonocclusive hygroscopic substances to the stratum corneum which will retain water, and make this water available to the stratum corneum to alter its physical properties and produce a cosmetically desirable effect.

The moisturizers herein are nonocclusive moisturizers. The preferred nonocclusives are liquid water-soluble polyols selected from glycerin, propylene glycol, sorbitol, polyethylene glycol, ethoxylated/propoxylated ethers of methyl glucose (e.g., methyl glyceth-20) and ethoxylated/propoxylated ethers of lanolin alcohol (e.g., Solulan®-75).

Other nonocclusive moisturizers are the compounds which are found to be naturally occurring in the stratum corneum of the skin selected from sodium pyrrolidone carboxylic acid, lactic acid, urea, L-proline, guanidine and pyrrolidone. Other water-soluble nonocclusive moisturisers are water-soluble hexadecyl, myristyl, isodecyl or isopropyl esters of adipic, lactic, oleic, stearic, isostearic, myristic or linoleic acids, sodium isostearoyl-2-lactylate, sodium capryl lactylate, hydrolyzed protein, aloe vera gel and acetamide MEA (N-acetylethanolamine).

The surfactant

The anionic and amphoteric surfactants used in this invention are selected from alkyl glyceryl ether sulfonates (AGS), anionic acyl sarcosinates, methyl acyl taurates, N-acyl glutamates, alkyl glucosides, acyl isethionates, alkyl sulfosuccinates, alkyl phosphate esters, ethoxylated alkyl phosphate esters, trideceth sulfate, methyl glucose esters, protein condensates, mixtures of ethoxylated alkyl sulfates and alkyl amine oxides, betaines, sultaines, and mixtures thereof. Alkyl chains for these surfactants are $C_8$—$C_{22}$, preferably $C_{10}$—$C_{18}$.

A preferred primary surfactant is sodium coco glyceryl ether sulfonate, which is mild and relatively nonirritating to the skin. This has been demonstrated in *in vitro* testing. While desirable to incorporate into a facial cleanser for its mildness properties, this coco AGS alone does not provide sufficient lather volume or speed of lather and produces a large, open bubble lather not of the rich, creamy small bubbled type desirable in facial cleansing. A 90/10 coconut/tallow alkyl distribution is most preferred. Certain secondary co-surfactants used in combination with AGS can provide a creamier and more stable foam. These secondary surfactants must also be intrinsically mild. Two secondary surfactants have been found to be especially desirable: a zwitterionic surfactant of the betaine class 70/30 laur/myristamidopropyl betaine (trade name Lexaine® LM, made by Inolex Corp.), and an anionic, sodium lauroyl sarcosinate (trade name Hamposyl® L, made by Hampshire Chemical).

Nonionics cannot be used as the sole surfactant in this product because of their low foaming ability; however, they can be incorporated as a co-surfactant. The amphoteric betaines and sultaines can be used as the sole surfactant, but are more preferred as a co-surfactant. Examples of betaines useful herein include the high alkyl betaines such as coco dimethyl carboxymethyl betaine, lauryl dimethyl carboxymethyl betaine, lauryl dimethyl alpha-carboxy-ethyl betaine, cetyl dimethyl carboxymethyl betaine, lauryl bis - (2 - hydroxy - propyl) carboxymethyl betaine, oleyl dimethyl gamma-carboxypropyl betaine, lauryl bis - (2 - hydroxypropyl) alpha-carboxyethyl betaine, etc. The sulfo betaines may be represented by coco sultaine, stearyl sultaine, lauryl dimethyl ethyl sulfobetaine, lauryl bis(2 - hydroxyethyl propyl sulfobetaine and like; amido betaines and amidosulfobetaines, wherein the RCONH $(CH_2)_3$ radical is attached to the nitrogen atom and are also useful in this invention.

It was also learned that the betaine employed in compositions of this invention (Lexaine® LM) can provide an additional benefit of soft/smooth skin feel through a desquamation effect, i.e., removal of dead skin cells (dry skin flakes) in in vitro testing. Although the zwitterionic surfactants of the betaine class (cetyl betaines like Lonzaine® 16S) are known to have this capability, generally it has been thought that the alkyl group chain length should be $C_{16}$ or higher. It was a surprising finding that the low levels of $C_{12}$—$C_{14}$ alkyl betaine employed in this development would provide a factor of 2× improvement in scale (i.e., dry skin flakes) removal vs. other facial cleansing products or a similar formulation not including the preferred betaine.

Therefore, a tri-component surfactant system of three surfactants employed in this invention (AGS, Lexaine® LM, sodium lauroyl sarcosinate) is highly desirable for the mousse of the present invention.

The ratios of AGS to co-surfactant are variable from 1:1 to 5:1, preferably 2:1 to 4:1. Also, three component mixtures of AGS/Lexaine LM/sarcosinate are believed desirable.

Other amphoterics of the betaine class are usable, i.e., cocamidopropyl betaine, cetyl betaine or sultaines.

Mixtures of ethoxylated alkyl sulfate and alkyl amine oxides have been found within certain ratios to provide mild surfactancy. A mole fraction of 0.5—1.0 amine oxide to alkyl sulfate is particularly mild as reported by K. Miyazawa et al., in *The International Journal of Cosmetic Science*, Vol. 6, (1984), pp. 33—46.

Other chain lengths ($C_{10}$—$C_{18}$) of any of the above mild surfactants or co-surfactants such as sodium myristoyl sarcosinate, etc., are also usable in this invention. Salts other than the sodium salt such as K-AGS and chain length distributions other than 90/10 coconut/tallow are usable.

The polymeric skin feel aids

The polymeric skin feel aids useful in the present invention are the cationic and the nonionic polymers used in the cosmetic field. Reduced skin irritation benefits of both types of polymers are set out in "Polymer JR for Skin Care" Bulletin, by Union Carbide, 1977. The cationics are preferred over the nonionic because they provide better skin feel benefits. Examples of the cationic polymers and the nonionic polymers useful in the present invention are set out below.

The amount of polymeric skin feel aid found useful in the concentrate is from 0.05% to 5%, preferably from 0.1% to 2%, and more preferably 0.1% to 1.0%.

In order to achieve superior after washing skin feel for this mousse, i.e., leaving the skin feeling less taut/more moisturized, softer and smoother, it was discovered that a combination of a high level of moisturizer (10—60% in the concentrate) and a selected polymeric ingredient, e.g., cationic (quaternized) guar gum (e.g., Jaguar® C-14-S), is required.

Other types of high molecular weight polymeric skin feel agents, such as nonionic guar gums, Merquats 100 and 550, made by Merck & Co., Inc; UCARE® Polymer JR-400, made by Union Carbide Corp.; Mirapol® A15 made by Miranol Chemical Company, Inc.; and Galactasol® 811, made by Henkel, Inc.; plus others, are usable. The polymer also provides enhanced creamy foam benefits.

The nonionic polymers found to be useful include the nonionic polysaccharides, e.g., nonionic

hydroxypropyl guar gums, offered by Celanese Water Soluble Polymers, a Division of Celanese Corp. A preferred nonionic hydroxypropyl guar gum material is Jaguar® HP-60 having molar substitution of about 0.6. Another class of useful nonionics is the cellulosic nonionic polymers, e.g., HEC and CMC.

The cationic polymers employed in this invention also provide a desirable silky, soft, smooth in-use feeling. The preferred level for this invention is 0.1—1% of the concentrate. While not being bound to any theory, it is believed that cationic polymers chemically interact with the anionic surfactants (e.g., AGS and sarcosinates) to form complexes which may enhance the mildness to skin characteristics of the already mild surfactants. Also, there is reason to believe that the positively charged cationic polymers can bond with negatively charged sites on the skin to provide a soft skin feel after use. Not to be bound by any theory, it is believed that the greater the charge density of the cationic polymer, the more effective it is for skin feel benefits.

One preferred cationic polymer is a cationically substituted galactomannan gum. The gum occurs naturally as guar gum, the principal component of the seed of the guar plant, *cyamopsis tetragonalobus*. The guar molecule is essentially a straight chain mannan branched at quite regular intervals with single membered galactose units on alternative mannose units. The mannose units are linked to each other by means of beta (1—4) glycosidic linkages. The galactose branching is accomplished through an alpha (1—6) linkage. The cationic derivatives are obtained by reaction between the hydroxyl groups of the polygalactomannan and reactive quaternary ammonium compounds. The degree of substitution "n" of the cationic groups is 0.11 to 0.22. The general formula for this cationic polymer is:

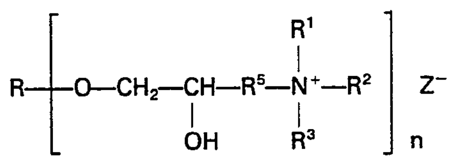

where R represents guar gum.

An example of a suitable quaternary ammonium derivative is hydroxypropyltrimethylammonium guar gum of the formula:

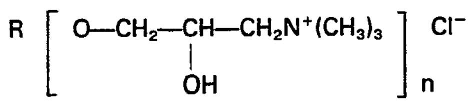

Such a material is available commercially from Celanese Water Soluble Polymers, a Division of Celanese Corp., Clifton, New Jersey 07012, under the trade name JAGUAR® C-14-S. This material also has the CTFA designation Guar Hydroxypropyltrimonium Chloride. Another suitable material is that known as JAGUAR® C-17 which is similar to JAGUAR® C-14-S, but has a higher degree of substitution of cationic groups. A further example of a suitable guar derivative is the hydroxypropylated cationic guar derivative known as JAGUAR® C-16 which, as well as containing the above cationic quaternary ammonium groups, also contains hydroxypropyl ($—CH_2CH(OH)CH_3$) substituent groups. JAGUAR® C-16 has a degree of substitution of the hydroxypropyl groups being 0.8—1.1.

Other suitable cationic polymers are copolymers of dimethylaminoethylmethacrylate and acrylamide and copolymers of dimethyldiallylammonium chloride and acrylamide in which the ratio of the cationic to neutral monomer units has been selected give a copolymer having a cationic charge.

A more complete list of cationic polymers useful in the present invention is described in US—A—4,438,095. Some of the more preferred cationics are listed in Col. 3, section 2; Col. 5, section 8; Col. 8, section 10; and Col. 9, lines 10—15 of the above reference.

The propellant

The propellants used in the compositions of the present invention are conventional materials, e.g., hydrocarbon and hydrocarbon mixtures, e.g., the mixture of butane, isobutane and propane, known commercially as Propellant A46, made by Phillips Chemical Co., a subsidiary of Phillips Petroleum Company, ethers and halohydrocarbons such as dimethyl ether or dichlorodifluoromethane (12) alone or mixtures thereof with dichlorotetrafluoroethane (114). Mixtures of hydrocarbon and halohydrocarbon propellants and nitrous oxide may also be used. The quantity of propellant used is in the range 3—12% by weight of the total emulsion. Preferred is 4—10% propellant.

Other ingredients

It is preferred that the cleansing product be formulated to provide a pH in use within the range of from 5 to 6, depending upon the particular surfactant or materials employed. Any of a large number of known substances can be used to adjust the pH of the liquid cleansing product, e.g., sodium hydroxide to raise the pH, citric acid to lower the pH, generally at a level of up to about 0.5% of the concentrate.

Emulsifiers may be added to the formula to improve the phase stability of the concentrate; however,

they are not necessary for a stable aerosol product that would be shaken before use—any slight phase separation is readily mixed into a homogeneous solution with only slight agitation.

Emulsifiers either alone or in combination with other formula ingredients, should be mild so as not to adversely affect the mildness of the total formula. Soaps, which are good emulsifiers, are acceptable at a level of 0.1—5% of the concentrate. An emulsifier such as PEG-600 is useful in the present invention at a level of 2—6% of the concentrate.

Other emulsifiers can be selected from the group consisting of polyethoxylated $C_8$—$C_{22}$ fatty acids having less than about 30 moles of ethylene oxide per mole of fatty acid, ethoxylated esters, unethoxylated sugar esters, polyoxyethylene fatty ether phosphates, fatty acid amides, phospholipids, polypropoxylated fatty ethers, acyl lactylates, polyethoxylated poly (oxypropylene) glycols, polypropoxylated poly (oxyethylene) glycols, poly (oxyethylene) poly (oxypropylene) ethylene diamines, and mixtures thereof.

Examples of such emulsifiers include polyoxyethylene (8) stearate, myristyl ethoxy (3) palmitate, methyl glucoside sesquistearate, sucrose distearate, sucrose laurate, sorbitan monolaurate, polyoxyethylene (3) oleyl ether phosphate, polyoxyethylene (10) oleyl ether phosphate, lauric diethanolamide, stearic monoethanolamide, lecithin, lanolin alcohol propoxylates, sodium stearoyl-2-lactylate, calcium stearoyl-2-lactylate and the Pluronics® offered by BASF Wyandotte.

Preferred emulsifiers are the polyethoxylated fatty acids having less than about 30 moles of ethylene oxide per mole of fatty acid, ethoxylated esters and the acyl lactylates.

It is also desirable to include a rinse aid in this product such as long chain length fatty acids (stearic acid), various clays, dry flow starch, Zn/Mg/Al stearate, calcium carbonate, precipitated amorphous silica, an amphoteric betaine or sultaine, or a selected nonionic surfactant. The preferred rinse aids are nonionic surfactants and fatty acids and mixtures thereof. The preferred nonionic surfactant rinse aid is a water-soluble polyoxyethylene derivative of a hydrophobic base, said derivative being a number of the group consisting of:

a. The reaction products of 9—20 carbon atom fatty acid monoesters of aliphatic polyhydric alcohols, which polyhydric alcohols contain at least 3 hydroxyls, with at least 10 moles of ethylene oxide;

b. The reaction products of 9—20 carbon atom alcohols, acids and mercaptans with at least two-thirds as many ethylene oxide units as the number of carbon atoms in the hydrophobic base, such as $AE_{12}$ and $C_{10}E_{10}$ ethoxylated anionics;

c. The reaction products of 12—24 carbon atoms alkylphenols and alkylcyclohexanols with at least as many ethylene oxide units as the number of carbon atoms in the hydrophobic base; and

d. Block copolymers of propylene oxide and ethylene oxide having the formula:

$$HO(CH_2CH_2O)_a(\underset{\displaystyle CH_3}{\underset{|}{CH}}\ CH_2O)_b(CH_2CH_2O)_cH$$

wherein a is an integer greater than seven, b is an integer from five to 20, and c is an integer, all such that (a plus c) is at least equal to b and is preferably at least twice b. This latter condition on the structure of useful block copolymers is designed to include only those which are sufficiently hydrophilic to give adequate stable foam. The useful block copolymers should also have a molecular weight from 1,000 to 20,000.

A preferred nonionic surfactant is polysorbate-20 (Tween® 20 made by ICI Americas, Inc.) which can be used in the mousse product of the present invention at a level of 1—10%, more preferably 3—8% of the concentrate.

Long chain saturated and unsaturated fatty acids, preferably stearic acid, can be used as a rinse aid in the mousse products of the present invention at a level of 0.25% to 10%, preferably 0.5% to 5% of the concentrate.

Fatty alcohols, $C_{10}$—$C_{18}$, preferably myristyl alcohol at a level of 0.05% to 5% of the concentrate, more preferably at about 0.1% can be used in the present invention.

Perfumes may be used in the cleansing products, generally at a level of 0.1% to 1% of the concentrate. Colorants may also be used. Opacifiers, e.g., ethylene glycol distearate, polystyrene latex, generally at a level of about 0.2% of the concentrate, may also be used to provide the mousse with an opaque or pearlescent appearance. Preservatives, e.g., EDTA, methyl paraben, propyl paraben, Germall® 115, Kathon®, generally at a level of less than 1% of the concentrate, may be incorporated in the emulsion to prevent microbiological growth.

Split face wash panel test (in vivo)

This test is conducted using expert female panelists who are sensitive to facial skin effect differences. The products were alternated between left and right sides of the face, as well as by order of the panelists.

The key question asked was "Which side of your face feels tighter?" Facial tautness is a key indicator of perceived mildness and the moisturization ability of a skin cleansing product.

The method

Mousse—Mousse test

1. Wet hands and face with water.
2. Shake can well before using.

3. Dispense a one inch puff (20—25 ml=0.8—1.25 g) nominally 1 g of mousse onto fingers or a wet wash cloth (do the same way as other product).

4. Apply the product to the appropriate side of face and rub gently, being careful not to get any product on the other half of face.

5. Rinse face with water, but do not dry.

Now, pat dry both sides of face with a paper towel. Wait 10 minutes, then answer the question "Which side of your face feels tighter?".

Bar—Mousse test

Same as Mousse—Mousse Test except one side of face is washed using the bar soap in usual manner (water only or with wash cloth).

Examples A—E

| Parts | | A | B | C | D | E |
|---|---|---|---|---|---|---|
| 94 | Concentrate | % | % | % | % | % |
| | AGS | 6 | 6 | 6 | 6 | 6 |
| | Hamposyl® L* | 2 | 2 | 2 | 2 | 2 |
| | Lauryl alcohol | 0.2 | 0.2 | 0.2 | — | — |
| | Glycerin | 15 | 20 | 5 | 15 | 5 |
| | Jaguar® C-14-S | 0.25 | 0.25 | — | 0.25 | — |
| | Stearic acid | 1 | 0.25 | 1 | — | — |
| | Tween® 20* | — | 4 | — | — | — |
| | Hydrolyzed protein | 0.45 | 0.45 | 0.45 | — | — |
| | Aloe vera gel | 1 | 1 | 1 | — | — |
| | PEG-600 | 4 | 4 | 4 | — | — |
| | Perfume | 0.2 | 0.2 | 0.2 | 0.2 | 0.2 |
| | Minors | 0.7 | 0.7 | 0.7 | 0.7 | 0.7 |
| | Water | Balance | Balance | Balance | Balance | Balance |
| | | | (Balance to 100 parts concentrate) | | | |
| | pH | 5—6 | 5—6 | 5—6 | 5—6 | 5—6 |
| 6 | Propellant A-46 | 6 | 6 | 6 | 6 | 6 |
| 100 | Parts | | | | | |

*Defined hereinabove.

TABLE 1
Split face wash panel test results
Examples A vs. C

| | |
|---|---|
| Less facial tautness | 84/14—statistically significant at a 99% confidence level |
| More smooth after-feel | 74/26—statistically significant at a 90% confidence level |

Examples D vs. E

| | |
|---|---|
| Less facial tautness | 64/36—statistically significant at a 95% confidence level |
| More smooth after-feel | 59/41—statistically significant at an 80% confidence level |

The above test results show that Example A of the present invention, with 15% glycerin and 0.25% polymer, is significantly milder than Example C, which has only 5% glycerin and no polymers, at confidence levels of 99% and 90%. Examples D vs. E compares the same base formulas as Examples A vs. C with the nonessential ingredients omitted.

Example A vs. DOVE® bar*

| | |
|---|---|
| Less facial tautness | 67/33—statistically significant at a 98% confidence level |
| Less redness | 59/41—statistically significant at an 80% confidence level |

The test of mousse Example A vs. DOVE® bar soap shows an advantage for the mousse product in terms of mildness and less irritation as measured by facial tautness and red appearance of the face after washing. The DOVE® bar is an accepted market-place mildness benchmark.

*The formula for DOVE® is estimated to be:

TABLE 2

| | |
|---|---|
| Na soap (T/Cn) | 11.7 (85/15) |
| Na cocoyl isethionate | 44.0 |
| Stearic acid | 26.0 |
| Water | 7.0 |
| Cn fatty acid | 2.5 |
| NaCl | 0.35 |
| Perfume | 1.25 |
| NaLAS | 1.80 |
| Na isethionate | 2.60 |
| Na stearate | 2.40 |
| $TiO_2$ | 0.40 |
| Total | 100.00 |

These data show that the mousse of this invention results in the desired mild skin cleansing action with improved skin feel leaving the skin feeling less taut and dry and more moisturized, softer and smoother.

**Claims**

1. A skin-cleansing emulsion mousse-forming product having a pressurized dispenser equipped with a dispensing head and valve and containing therein a foam-forming emulsion comprising:

a. 88% to 97% by weight of the emulsion of a concentrate containing by weight of the concentrate:

(1). from 3% to 20% of a nonsoap surfactant selected from alkyl glyceryl ether sulfonates (AGS), anionic acyl sarcosinates, methyl acyl taurates, N-acyl glutamates, alkyl glucosides, acyl isethionates, alkyl sulfosuccinates, alkyl phosphate esters, ethoxylated alkyl phosphate esters, trideceth sulfate, methyl glucose esters, protein condensates, mixtures of ethoxylated alkyl sulfates and alkyl amine oxides, betaines, sultaines, and mixtures thereof;

(2). a polymeric skin feel aid at a level of from 0.05% to 5%;

(3). a nonocclusive moisturizer at a level of from 10% to 60%, the moisturizer being selected from:

a) water-soluble liquid polyols, said polyols being selected from glycerin, polyethylene glycol, propylene glycol, sorbitol, polyethylene glycol and propylene glycol ethers of methyl glucose, polyethylene glycol and propylene glycol ethers of lanolin alcohol, and mixtures thereof;

b) essential compounds found naturally occurring in the stratum corneum of the skin, said essential compounds being selected from sodium pyrrolidone carboxylic acid, lactice acid, urea, L-proline, guanidine, pyrrolidone, and mixtures thereof;

c) water-soluble nonpolyol nonocclusives selected from hexadecyl, myristyl, isodecyl or isopropyl esters of adipic, lactic, oleic, stearic, isostearic, myristic or linoleic acids, sodium isostearoyl-2-lactylate, sodium capryl lactylate, hydrolyzed protein, aloe vera gel and acetamide MEA (N-acetylethanolamine), and mixtures thereof;

(4). water; and

b. from 3% to 12% by weight of the emulsion of a propellant.

2. The skin-cleansing emulsion mousse-forming product of Claim 1 wherein the moisturizer is selected from said essential compounds found naturally occurring in the stratum corneum of the skin and said liquid polyols.

3. The skin-cleansing emulsion mousse-forming product of Claim 1 wherein said moisturizer is glycerin.

4. The skin-cleansing emulsion mousse-forming product of Claim 1 wherein said moisturizer is present at a level of 12% to 40% by weight of the concentrate.

5. The skin-cleansing emulsion mousse-forming product of Claim 1 wherein said polymer is selected from cationic and nonionic polysaccharides; cationic and nonionic homopolymers and copolymers derived from acrylic and/or methacrylic acid; cationic and nonionic cellulosic resins; cationic copolymers of dimethyldiallylammonium chloride and acrylic acid; cationic homopolymers of dimethyldiallylammonium chloride; cationic polyalkylene and ethoxypolyalkylene imines, the mixtures thereof.

6. The skin-cleansing emulsion mousse-forming product of Claim 5 wherein said polymer is a cationic polymer.

7. The skin-cleansing emulsion mousse-forming product of Claim 5 wherein said polymer is a cationic guar gum.

8. The skin-cleansing emulsion mousse-forming product of Claim 1 wherein said polymer is at 0.1% to 2% by weight and said glycerin is at 12% to 40% by weight of the concentrate.

9. The skin-cleansing emulsion mousse-forming product of Claim 1 wherein said surfactant is present at a level of 4% to 15% by weight of the concentrate.

10. The skin-cleansing emulsion mousse-forming product of Claim 1 wherein said surfactant is present at a level of 6% to 12% of the concentrate.

11. The skin-cleansing emulsion mousse-forming product of Claim 1 wherein said surfactant is an AGS/sarcosinate mix having a weight ratio of 1:1 to 5:1.

12. The skin-cleansing emulsion mousse-forming product of Claim 11 wherein said AGS/sarcosinate has a weight ratio of 2:1 to 4:1.

13. The skin-cleansing emulsion mousse-forming product of Claim 1 wherein said concentrate also contains a co-surfactant selected from nonionic, amphoteric betaine or amphoteric sultaine, and wherein the surfactant and the co-surfactant have a weight ratio of 1:1 to 5:1.

14. The skin-cleansing emulsion mousse-forming product of Claim 13 wherein said concentrate contains from 1% to 10%, preferably from 3% to 8% by weight of a nonionic surfactant.

15. The skin-cleansing emulsion mousse-forming product of Claim 1 wherein said concentrate contains from 0.25% to 10%, preferably from 0.5% to 5% by weight of stearic acid.

16. The skin-cleansing emulsion mousse-forming product of Claim 1 wherein said concentrate contains fatty alcohol at a level of from 0.05% to 5%, preferably from 0.05% to 1% by weight.

17. The skin-cleansing emulsion mousse-forming product of Claim 1 wherein said polymer is at 0.1% to 1% by weight; said glycerin is at 15% to 40% by weight; and said concentrate contains stearic acid at 0.5% to 5% by weight, and $C_{10}$—$C_{18}$ fatty alcohol at 0.1% by weight.

18. The skin-cleansing emulsion mousse-forming product of Claim 1 wherein said concentrate contains from 2% to 6% by weight of PEG-600.

19. The skin-cleansing emulsion mousse-forming product of Claim 1 wherein said concentrate contains from 0.1% to 5% by weight of a hydrolyzed protein.

20. The skin-cleansing emulsion mousse-forming product of Claim 1 wherein said concentrate contains from 0.5% to 5% by weight of aloe vera gel.

21. The skin-cleansing emulsion mousse-forming product of Claim 1 wherein said concentrate has a pH of from 5 to 6.

**Patentansprüche**

1. Hautreinigenden Emulsionsschaum bildendes Produkt, welches ein mit einem Abgabekopf und einem Ventil ausgerüstetes, unter Druck gesetztes Abgabemittel besitzt und darin eine schaumbildende Emulsion enthält, umfassend:

a. 88 Gew.-% bis 97 Gew.-% der Emulsion an einem Konzentrat, das, bezogen auf das Gewicht des Konzentrats:

(1). von 3% bis 20% eines Nichtseifen-artigen grenzflächenaktiven Mittels, ausgewählt unter Alkylglycerylethersulfonaten (AGS), anionischen Acylsarcosinaten, Methylacyltauraten, N - Acylglutamaten, Alkylglucosiden, Acylisethionaten, Alkylsulfosuccinaten, Alkylphosphatestern, ethoxylierten Alkylphosphatestern, Tridecethsulfat, Methylglucoseestern, Proteinkondensaten, Gemischen aus ethoxylierten Alkylsulfaten und Alkylaminoxiden, Betainen, Sultainen und Gemischen hievon;

(2). einen polymeren Hilfsstoff für das Anfühlen der Haut in einer Menge von 0,05% bis 5%;

(3). ein nicht-verschließendes Feuchtigkeitsmittel in einer Menge von 10% bis 60%, wobei das Feuchtigkeitsmittel ausgewählt ist unter:

a) wasserlöslichen, flüssigen Polyolen, welche Polyole unter Glycerin, Polyethylenglykol, -propylenglykol, Sorbit, Polyethylenglykol- und -propylenglykolethern von Methylglucose, Polyethylenglykol- und -propylenglykolethern von Lanolinalkohol und Gemischen hievon ausgewählt sind;

b) essentiellen Verbindungen, welche im Stratum corneum der Haut natürlich vorkommend gefunden werden, welche essentiellen Verbindungen unter Natriumpyrrolidoncarbonsäure, Milchsäure, Harnstoff, L-Prolin, Guanidin, Pyrrolidon und Gemischen hievon ausgewählt sind;

c) wasserlöslichen, nicht-verschließenden Nicht-Polyolverbindungen, ausgewählt unter Hexadecyl-, Myristyl-, Isodecyl- oder Isopropylestern von Adipinsäure, Milchsäure, Ölsäure, Stearinsäure, Isostearinsäure, Myristinsäure oder Linolsäure, Natriumisostearoyl - 2 - lactylat, Natriumcapryllactylat, hydrolysiertem Protein, Aloe-vera-Gel und Acetamid-MEA (N-Acetylethanolamin) und Gemischen hievon;

(4). Wasser; enthält, und

b. von 3 Gew.-% bis 12 Gew.-% der Emulsion an einem Treibmittel.

2. Hautreinigenden Emulsionsschaum bildendes Produkt nach Anspruch 1, worin das Feuchtigkeitsmittel unter den genannten essentiellen Verbindungen, welche im Stratum corneum der Haut natürlich vorkommend gefunden werden, und den genannten flüssigen Polyolen ausgewählt ist.

3. Hautreinigenden Emulsionsschaum bildendes Produkt nach Anspruch 1, worin das genannte Feuchtigkeitsmittel Glycerin ist.

4. Hautreinigenden Emulsionsschaum bildendes Produkt nach Anspruch 1, worin das genannte Feuchtigkeitsmittel in einer Menge von 12 Gew.-% bis 40 Gew.-% des Konzentrats vorliegt.

5. Hautreinigenden Emulsionsschaum bildendes Produkt nach Anspruch 1, worin das genannte Polymer unter kationischen und nichtionischen Polysacchariden; sich aus Acryl- und/oder Methacrylsäure herleitenden kationischen und nichtionischen Homopolymeren und Copolymeren; kationischen und nichtionischen Celluloseharzen; kationischen Copolymeren von Dimethyldiallylammoniumchlorid und Acrylsäure; kationischen Homopolymeren von Dimethyldiallylammoniumchlorid; kationischen Polyalkylen- und Ethoxypolyalkyleniminen und Gemischen hievon ausgewählt ist.

6. Hautreinigenden Emulsionsschaum bildendes Produkt nach Anspruch 5, worin das genannte Polymer ein kationisches Polymer ist.

7. Hautreinigenden Emulsionsschaum bildendes Produkt nach Anspruch 5, worin das genannte Polymer ein kationischer Guargummi ist.

8. Hautreinigenden Emulsionsschaum bildendes Produkt nach Anspruch 1, worin das genannte Polymer in einer Menge von 0,1 Gew.-% bis 2 Gew.-% des Konzentrats vorliegt und das genannte Glycerin in einer Menge von 12 Gew.-% bis 40 Gew.-% des Konzentrats vorliegt.

9. Hautreinigenden Emulsionsschaum bildendes Produkt nach Anspruch 1, worin das genannte grenzflächenaktive Mittel in einer Menge von 4 Gew.-% bis 15 Gew.-% des Konzentrats vorliegt.

10. Hautreinigenden Emulsionsschaum bildendes Gemisch nach Anspruch 1, worin das genannte grenzflächenaktive Mittel in einer Menge von 6% bis 12% des Konzentrats vorliegt.

11. Hautreinigenden Emulsionsschaum bildendes Produkt nach Anspruch 1, worin das genannte grenzflächenaktive Mittel ein AGS/Sarcosinat-Gemisch mit einem Gewichtsverhältnis von 1:1 bis 5:1 ist.

12. Hautreinigenden Emulsionsschaum bildendes Produkt nach Anspruch 11, worin das genannte AGS/Sarcosinat ein Gewichtsverhältnis von 2:1 bis 4:1 aufweist.

13. Hautreinigenden Emulsionsschaum bildendes Produkt nach Anspruch 1, worin das genannte Konzentrat auch ein co-grenzflächenaktives Mittel, ausgewählt unter nichtionischem, amphoterem Betain oder amphoterem Sultain, enthält, und worin das grenzflächenaktive Mittel und das co-grenzflächenaktive Mittel ein Gewichtsverhältnis von 1:1 bis 5:1 aufweisen.

14. Hautreinigenden Emulsionsschaum bildendes Produkt nach Anspruch 13, worin das genannte Konzentrat von 1 Gew.-% bis 10 Gew.-%, vorzugsweise von 3 Gew.-% bis 8 Gew.-%, eines nichtionischen grenzflächenaktiven Mittels enthält.

15. Hautreinigenden Emulsionsschaum bildendes Produkt nach Anspruch 1, worin das genannte Konzentrat von 0,25 Gew.-% bis 10 Gew.-%, vorzugsweise von 0,5 Gew.-% bis 5 Gew.-%, Stearinsäure enthält.

16. Hautreinigenden Emulsionsschaum bildendes Produkt nach Anspruch 1, worin das genannte Konzentrat Fettalkohol in einer Menge von 0,05 Gew.-% bis 5 Gew.-%, vorzugsweise von 0,05 Gew.-% bis 1 Gew.-%, enthält.

17. Hautreinigenden Emulsionsschaum bildendes Produkt nach Anspruch 1, worin das genannte Polymer in einer Menge von 0,1 Gew.-% bis 1 Gew.-% vorliegt; das genannte Glycerin in einer Menge von 15 Gew.-% bis 40 Gew.-% vorliegt; und das genannte Konzentrat Stearinsäure in einer Menge von 0,5 Gew.-% bis 5 Gew.-% und $C_{10}$—$C_{18}$-Fettalkohol in einer Menge von 0,1 Gew.-% enthält.

18. Hautreinigenden Emulsionsschaum bildendes Produkt nach Anspruch 1, worin das genannte Konzentrat von 2 Gew.-% bis 6 Gew.-% PEG-600 enthält.

19. Hautreinigenden Emulsionsschaum bildendes Produkt nach Anspruch 1, worin das genannte Konzentrat von 0,1 Gew.-% bis 5 Gew.-% eines hydrolysierten Proteins enthält.

20. Hautreinigenden Emulsionsschaum bildendes Produkt nach Anspruch 1, worin das genannte Konzentrat von 0,5 Gew.-% bis 5 Gew.-% Aloe-vera-Gel enthält.

21. Hautreinigenden Emulsionsschaum bildendes Produkt nach Anspruch 1, worin das genannte Konzentrat einen pH-Wert von 5 bis 6 besitzt.

**Revendications**

1. Un produit en émulsion formant mousse pour le nettoyage de la peau comprenant un distributeur pressurisé équipé d'une tête de distribution et d'une valve et contenant une émulsion formant mousse qui comprend:

a. 88% à 97% en poids, par rapport à l'émulsion, d'un concentré contenant, en poids du concentré:

(1). 3% à 20% d'un agent de surface sans savon choisi parmi les alkylglycéryl éther sulfonates (AGS), les acylsarcosinates anioniques, les méthyl acyltaurates, les N-acylglutamates, les alkylglucosides, les acyliséthionates, les alkylsulfosuccinates, les esters alkylphosphoriques, les esters alkylphosphoriques

éthoxylés, le tridéceth sulfate, les esters de méthylglucose, les produits de condensation de protéines, les mélanges d'alkylsulfates éthoxylés et d'oxydes d'alkylamines, les bétaïnes, les sultaïnes et leurs mélanges;

(2). un produit polymère adoucissant la peau à la dose de 0,05 à 5%;

(3). un hydratant non occlusif à la dose de 10% à 60%, l'hydratant étant choisi parmi:

a) des polyols liquides hydrosolubles, lesdits polyols étant sélectionnées parmi la glycérine, le polyéthylèneglycol, le propylèneglycol, le sorbitol, les éthers de polyéthylèneglycol et de propylèneglycol du méthylglucose, les éthers de polyéthylèneglycol et de propylèneglycol d'alcool de lanoline et leurs mélanges;

b) les composés essentiels se trouvant à l'état naturel dans la couche cornée de la peau, lesdits composés essentiels étant choisis parmi le pyrrolidone carboxylate de sodium, l'acide lactique, l'urée, la L-proline, la guanidine, la pyrrolidone et leurs mélanges;

c) les non occlusifs non polyols hydrosolubles choisis parmi les esters hexadécyliques, myristyliques, isodécyliques ou isopropyliques des acides adipique, lactique, oléique, stéarique, isostéarique, myristique ou linoléique, l'isostéaroyl-2-lactylate de sodium, le capryl lactylate de sodium, les protéines hydrolysées, le gel d'aloe vera et l'acétamide MEA (N-acétyléthanolamine) et leurs mélanges;

(4). de l'eau; et

b. 3% à 12% en poids, par rapport à l'émulsion, d'un propulseur.

2. Le produit en émulsion formant mousse pour le nettoyage de la peau selon la revendication 1, dans lequel l'hydratant est choisi parmi lesdits composés essentiels se trouvant à l'état naturel dans la couche cornée de la peau et lesdits polyols liquides.

3. Le produit en émulsion formant mousse pour le nettoyage de la peau selon la revendication 1, dans lequel ledit hydratant est la glycérine.

4. Le produit en émulsion formant mousse pour le nettoyage de la peau selon la revendication 1, dans lequel ledit hydratant est présent à la dose de 12% à 40% en poids du concentré.

5. Le produit en émulsion formant mousse pour le nettoyage de la peau selon la revendication 1, dans lequel ledit polymère est sélectionné parmi les polysaccharides cationiques et non ioniques; les homopolymères et copolymères cationiques et non ioniques dérivés de l'acide acrylique et/ou méthacrylique; les résines cellulosiques cationiques et non ioniques; les copolymères cationiques de chlorure de diméthyldiallylammonium et d'acide acrylique; les homopolymères cationiques du chlorure de diméthyldiallylammonium; les polyalkylène et éthoxypolyalkylèneimines cationiques et leurs mélanges.

6. Le produit en émulsion formant mousse pour le nettoyage de la peau selon la revendication 5, dans lequel ledit polymère est un polymère cationique.

7. Le produit en émulsion formant mousse pour le nettoyage de la peau selon la revendication 5, dans lequel ledit polymère est une gomme guar cationique.

8. Le produit en émulsion formant mousse pour le nettoyage de la peau selon la revendication 1, dans lequel ledit polymère représente 0,1% à 2% en poids et ladite glycérine représente 12% à 40% en poids du concentré.

9. Le produit en émulsion formant mousse pour le nettoyage de la peau selon la revendication 1, dans lequel ledit agent de surface est présent à la dose de 4% à 15% en poids du concentré.

10. Le produit en émulsion formant mousse pour le nettoyage de la peau selon la revendication 1, dans lequel ledit agent de surface est présent à la dose de 6% à 12% du concentré.

11. Le produit en émulsion formant mousse pour le nettoyage de la peau selon la revendication 1, dans lequel ledit agent de surface est un mélange AGS/sarcosinate dans un rapport pondéral de 1:1 à 5:1.

12. Le produit en émulsion formant mousse pour le nettoyage de la peau selon la revendication 11, dans lequel ledit mélange AGS/sarcosinate présente un rapport pondéral de 2:1 à 4:1.

13. Le produit en émulsion formant mousse pour le nettoyage de la peau selon la revendication 1, dans lequel ledit concentré contient également un co-surfactif choisi parmi un non ionique, une bétaïne amphotère ou une sultaïne amphotère et dans lequel l'agent de surface et le co-surfactif présentent un rapport pondéral de 1:1 à 5:1.

14. Le produit en émulsion formant mousse pour le nettoyage de la peau selon la revendication 13, dans lequel ledit concentré contient 1% à 10%, de préférence 3% à 8% en poids d'un agent de surface non ionique.

15. Le produit en émulsion formant mousse pour le nettoyage de la peau selon la revendication 1, dans lequel ledit concentré contient 0,25% à 10%, de préférence 0,5% à 5% en poids d'acide stéarique.

16. Le produit en émulsion formant mousse pour le nettoyage de la peau selon la revendication 1, dans lequel ledit concentré contient de l'alcool gras à la dose de 0,05% à 5%, de préférence de 0,05% à 1% en poids.

17. Le produit en émulsion formant mousse pour le nettoyage de la peau selon la revendication 1, dans lequel ledit polymère est présent à raison de 0,1% à 1% en poids; ladite glycérine est présente à raison de 15% à 40% en poids et ledit concentré contient de l'acide stéarique à raison de 0,5% à 5% en poids et de l'alcool gras en $C_{10}$ à $C_{18}$ à raison de 0,1% en poids.

18. Le produit en émulsion formant mousse pour le nettoyage de la peau selon la revendication 1, dans lequel ledit concentré contient 2% à 6% en poids de PEG-600.

19. Le produit en émulsion formant mousse pour le nettoyage de la peau selon la revendication 1, dans lequel ledit concentré contient 0,1% à 5% en poids d'une protéine hydrolysée.

20. Le produit en émulsion formant mousse pour le nettoyage de la peau selon la revendication 1, dans lequel ledit concentré contient 0,5% à 5% en poids de gel d'aloe vera.

21. Le produit en émulsion formant mousse pour le nettoyage de la peau selon la revendication 1, dans lequel ledit concentré possède un pH de 5 à 6.